# EUROPEAN PATENT SPECIFICATION

(11) **EP 1 225 943 B1**
(45) Date of publication and mention of the grant of the patent: **24.01.2007**
(21) Application number: 00990467.3
(22) Date of filing: 01.11.2000
(51) Int. Cl.: A61M 15/00

(54) **INTERNAL VORTEX MECHANISM FOR INHALER DEVICE**
INTERNE VERWIRBELUNGSVORRICHTUNG FÜR EIN INHALIERGERÄT
MECANISME INTERNE DE TOURBILLON POUR DISPOSITIF D'INHALATION

(30) Priority: 01.11.1999 US 431266
(43) Date of publication of application: 31.07.2002
(73) Proprietor: Systemic Pulmonary Delivery, Ltd., St. Louis, MO 63017 (US)
(72) Inventor: GENOSAR, Amir, 37000 Pardess-Hana (IL); PAVKOV, Richard, M., Plymouth, MI 48170 (US)
(74) Representative: Rupp, Christian
(86) International application number: PCT/US2000/041722
(87) International publication number: WO 2001/036033

(56) References cited:
- EP-A- 0 911 048
- WO-A-93/00951
- US-A- 5 676 130

## Description

### Field of the Invention

The present invention relates to methods and apparatuses for delivering a dose of aerosolized medication by inhalation into the lungs of a patient, and more particularly to an internal vortex mechanism for an inhaler device.

### Background of the Invention

Aerosols are increasingly being used for delivering medication into the lungs for therapeutic treatment of the body. For example, in the treatment of asthma, inhalers are commonly used for delivering bronchodilators such as β₂ agonists and anti-inflammatory agents such as corticosteroids. Two types of inhalers are in common usemetered dose inhalers (MDIs) and dry powder inhalers (DPIs).

In a conventional MDI device, the medication is provided by the pharmaceutical manufacturer in a pressurized aerosol canister, with the medication being suspended or dissolved in a liquid propellant such as a chloro fluorocarbon (CFC) or hydrofluoroalkane (HFA). The canister includes a metering valve having a hollow discharge stem which can be depressed inward into the canister to discharge a metered volume of propellant-medication mixture in the form of an aerosol comprising fine droplets of propellant in which particles of the medication are suspended or dissolved.

A conventional MDI for use with such a canister includes a housing having an actuator and nozzle. The canister is inserted into the housing with the hollow discharge stem of the canister being received in a bore in the actuator. Depressing the closed end of the canister causes the stem to be pushed inward into the canister so that a metered volume of medication is discharged through the nozzle. The housing further defines a flowpath in fluid communication with the nozzle, the flowpath having an outlet at a mouthpiece portion of the housing, such that the aerosolized medication may be inhaled after it exits the mouthpiece portion. The patient either inserts the mouthpiece into the mouth with the lips closed around the mouthpiece, or holds the mouthpiece at a slight distance away from an open mouth.
The patient then depresses the canister to discharge the medication, and simultaneously inhales.

While generally good for many applications, existing MDIs suffer from a number of significant disadvantages. One problem with existing MDIs is poor delivery efficiency of the medication. It has been estimated that on average, with existing MDIs, only about 10 percent of the medication dose which is dispensed from the canister actually reaches the lungs where it can achieve the intended result. A significant portion of the medication impacts and sticks to the inner surfaces of the MDI device. This makes MDIs less than optimal for delivering expensive medication.

An MDI is disclosed in EP 0 911 048 A1. The MDI includes a housing defining a conduit with a mouthpiece, and an actuator with a nozzle discharge orifice arranged to discharge aerosol into the conduit. An air tube is arranged within the conduit with an inlet and an outlet. Auxiliary air inlets in the closed end of the conduit provide a boundary layer flow along the inner wall of the conduit. Vortex generators on the inner wall of the conduit add vorticity and turbulence to the boundary layer flow.

Accordingly, it is an object of the present invention to provide an apparatus for delivering an aerosolized medication in which the respirable fraction of the metered dose (i. e., the fraction in the form of particles of the optimum size) is maximized at the exit of the apparatus.

Another object of the invention is to provide an apparatus for delivering an aerosolized medication in which impaction and sticking of medication on the inner walls of the apparatus is minimized.

Yet another object of the present invention is to provide an MDI device with a circumferential-swirling turbulent boundary layer of air along the inner surface of the MDI device in a cost effective and simple manner.

### Summary of the Invention

The above and other objects of the invention are achieved by the apparatus of the invention that follows, in which flow control techniques and devices are used primarily to limit impaction and sticking of medication to the inner surfaces of an MDI device, and also aid in:
- mixing ambient air with the mediation and help disperse the plume of aerosolized medication;
- evaporating the aerosol propellent of the medication;
- directing the air/medication mixture to the mouthpiece for inhalation by a patient.

In one embodiment of the invention, an aerosol flow control apparatus includes a housing having an open end and a conduit with a wall including an inner surface, a medication dispenser disposed within or supported in the housing and adapted to dispense a dose of aerosolized medication into the conduit, and a plurality of vortex generators positioned downstream from the medication dispenser. The vortex generators establish a circumferential-swirling turbulent air flow along the inner surface upon an air flow being established through the open end.

The apparatus also includes a plurality of air inlets in fluid communication with ambient outside air.

The vortex generators are positioned within the wall and have a plurality of outlets located on the inner surface, wherein the vortex generators are substantially pyramid-shaped having an open base forming said outlet and having an apex in fluid communication with the air inlets.

One of the unique features of the present invention is the decreased cost of MDI devices using the vortex generators according to the present invention, as well as the simplification of their manufacture. Specifically, the unique pyramid shapes of the vortex generators are inexpensive to produce and allow for easy fabrication/modification in plastic molding. By molding the vortex generators within the wall of the conduit, the cost of the extra material when molding vanes on the inner wall of the conduit is eliminated. In addition, the complexity of the mold for producing the conduit is decreased allowing easier manufacture of the mold and easier production of the MDI device.

The present invention results in easy manufacture using plastic injection molding, where the unique vortex generator design allows for easy pull for fabrication in plastic.

These and other objects and advantages of the present invention shall become more apparent from the accompanying drawings and the description thereof.

### Brief Description of the Drawings

The accompanying drawings, which are incorporated in and constitute a part of this specification, illustrate various embodiments of the invention and, together with the general description of the invention given above and the detailed description given below, serve to explain the principles of the invention.

Fig. 1 is a side, sectional view of the present invention.

Fig. 2A is a perspective view of the rear wall of the housing of the MDI device according to the present invention, illustrating the auxiliary air inlets and a portion of the vortex generators.

Fig. 2B is an enlarged view of an auxiliary air inlet for the MDI device according to the present invention illustrating one of the pyramid shaped portions for the vortex generators.

Fig. 3 is a perspective view of the front portion of the rear wall of the housing of the MDI device according to the present invention

Fig. 4A is a side, section view of the rear wall of the housing of the MDI device according to the present invention illustrating the location of the vortex generators outlets.

Fig. 4B is a side, sectional view of the vortex generators according to the present invention, illustrating the overlapping apexes of first and second pyramid shaped portions.

Detailed Description of the Drawings

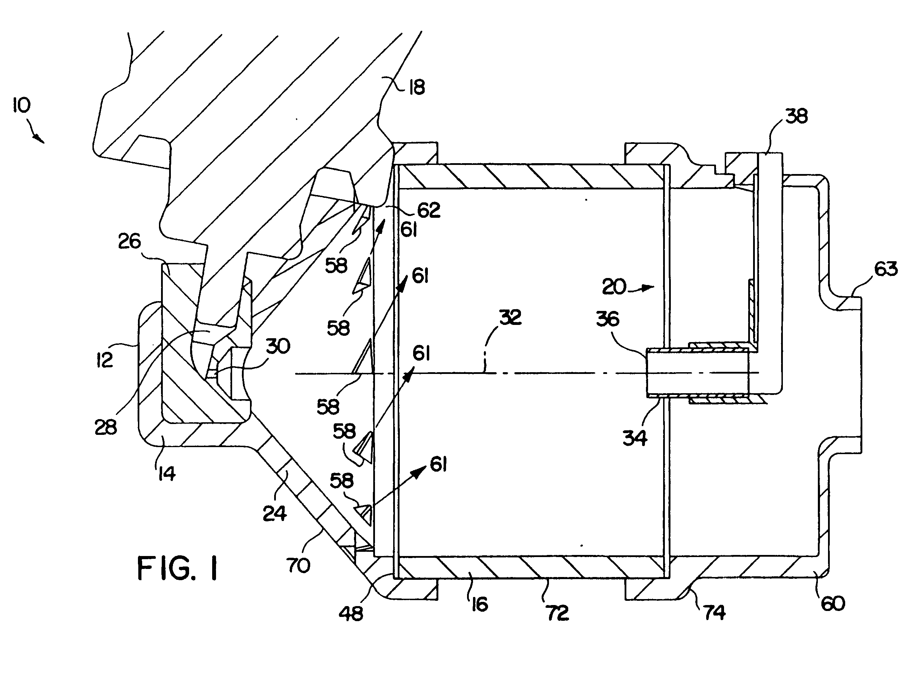

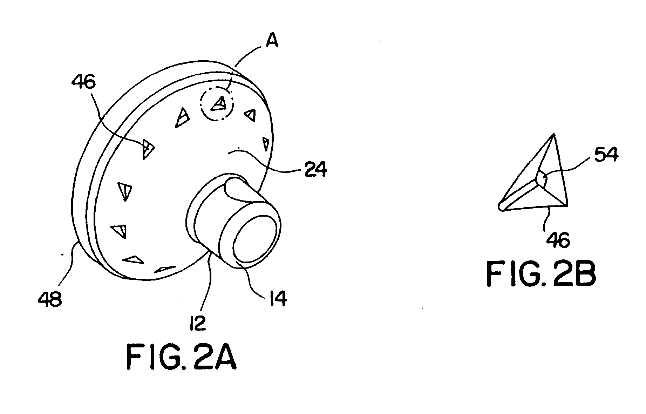

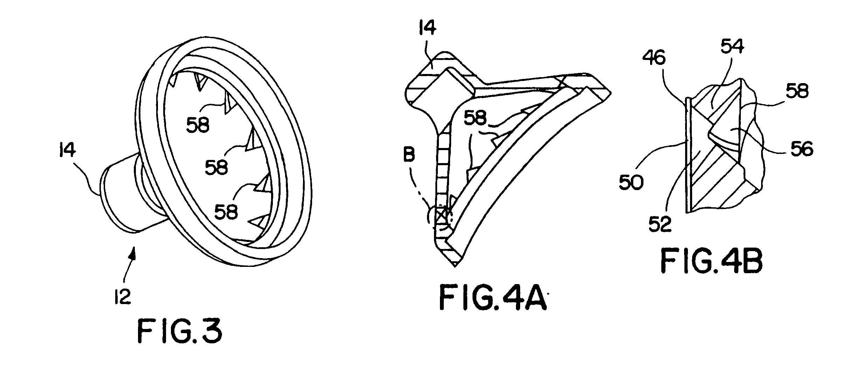

FIGS. 1-4B depict an inhaler in accordance with the principles of the invention. The inhaler includes a housing 12 which has a receptacle portion 14 connected to a conduit 16.

The inhaler apparatus of the present invention is usable with any standard pressurized canister having an internal metering valve with a hollow discharge stem which may be depressed inwardly with respect to the canister body from an inoperative position in which discharge of medication is prevented, to an operative position in which a metered volume of the canister contents is discharged through the hollow discharge stem.

The conduit 16 includes an open end 20 spaced from the receptacle portion 14, and a closed end defined by an end wall 24 which is connected to the receptacle portion 14.

The end wall 24 preferably is generally conical or hemispherical in shape, with an apex of the end wall 24 forming the portion of the end wall 24 farthest from the open end 20.

The inhaler 10 also includes a separate mouthpiece 60 which connects to the open end 20 of the conduit 16. The mouthpiece 60 has a reduced diameter portion 63 adapted to be inserted into the mouth of a user. The mouthpiece 60 also includes an air tube 34, having an outlet 36 spaced downstream of and in opposing relationship with the nozzle discharge orifice 30, and an air inlet 38 located on the outside of the mouthpiece. The air inlet 38 is in fluid communication with the outlet 36 of the air tube 34, providing ambient outside air to impinge on a plume of medication discharged from the orifice 30. providing ambient outside air to impinge on a plume of medication discharged from the orifice 30.

The housing 12 further includes an actuator and nozzle assembly 26 supported by the end wall 24. The actuator and nozzle assembly 26 includes a bore 28 which is adapted to receive the hollow discharge stem of the canister 18, and a nozzle discharge orifice 30 in fluid communication with the bore 28. The nozzle discharge orifice 30 is advantageously located at the apex of the end wall 24 and oriented to direct an aerosol plume generally along the central longitudinal axis 32 of the conduit. The orifice 30 preferably has an internal diameter at the exit of less than about 0.635 mm (0.025 inch), and more preferably between about 0.127 mm (0.005 inch) and about 0.483 mm (0.019 inch).

The housing 12 may be formed in three sections as shown in Fig. 1. However, for ease of manufacturing, the housing 12 may alternatively be formed in fewer than three sections. For example, the housing 12 may be formed in two sections, a first section including the receptacle portion 14, end wall 24, and the conduit 16, and a second section including the mouthpiece 60. Alternatively, the housing 14 may be formed in two split sections, split on a longitudinal plane through the conduit, where the two sections being generally mirror images of each other and are joined together along the plane of symmetry. Nevertheless, for illustration purposes, an embodiment having three sections is shown and described.

A first section 70 includes the receptacle portion 14, the end wall 24 and actuator and nozzle assembly 26, and a generally cylindrical portion 62 which forms a part of the conduit 16 and is connected to the end wall 24 at the juncture 48. The first section 70 advantageously is integrally formed of one piece, although it may alternatively be formed in multiple pieces which are subsequently joined together.

A second section 72 includes a second generally cylindrical portion whose inner and outer diameters are less than those of the first generally cylindrical section 70. The reduced diameter allows the second section to be received within the open end of the first section at the juncture 48. Second section 72 preferably is integrally formed in one piece, although it may alternatively be formed in multiple pieces which are subsequently joined.

A third section 74 of the housing 12 includes the mouthpiece 60, which is generally cylindrical and receives the other end (open end 20) of the second section 72, and also includes the air tube 34 with corresponding air inlet 36 located on the outer surface of the MDI device and the outlet 38 which provides the impinging jet.

Although the present invention is illustrated having a second section with a diameter less than that of the first section and the third section (mouthpiece), any design that allows for the connection of the sections may be used.

The housing 12 advantageously is formed of a plastic such as polyamide, polyester, polypropylene, polyethylene, ABS, polycarbonate, or polyacrylate. The housing 12 may be manufactured by any suitable technique such as injection molding or blow molding.

The inhaler 10 also includes a plurality of vortex generators within the wall of the conduit adjacent the juncture between the end wall and the conduit, and more preferably, on the end wall adjacent the juncture. Each vortex generator includes a first pyramid shaped portion 56 having an open base 58 forming an outlet on the inner surface of the conduit/end wall, and having an apex which is in fluid communication with a corresponding auxiliary ambient air inlet 46. The auxiliary air inlets 46 may be positioned on the outer surface of the end wall 24, circumferentially spaced therearound, and preferably located adjacent the juncture 48.

Preferably, the vortex generators also include a second pyramid shape, substantially similar to the first pyramid shape, having an open base in fluid communication with the auxiliary air inlets (or forming the auxiliary air inlets). The second pyramid portion includes an apex which overlaps the apex of the first pyramid portion, establishing fluid communication and creating overlapping portion 54 between the pyramid portions.

The pyramid shapes of the vortex generators yield an air flow 61 having radial and axial components relative to the conduit, establishing a circumferential-swirling, turbulent boundary layer along the inside of the conduit. This boundary layer primarily reduces the likelihood of medication particles impacting and permanently sticking to the inner walls of the conduit, and also aids in mixing air with the medication, evaporating the aerosol propellent, and directing the air/medication mixture to the mouthpiece.

Each pyramid shape includes one side at a 90 degree angle to the bottom surface. Using two pyramid shapes for the vortex generators, the apexes of the pyramids are formed together on the 90 degree sides to create an overlap (a shut-off), establishing an opening between the two pyramid shapes. The size of the opening can be varied by increasing or decreasing the apex overlap between the two pyramids. The larger the overlap, the larger the opening. Ultimately, the size of the opening determines the amount of air flow over the vortex generators. Thus, controlling the overlap determines the amount of auxiliary air flow in the boundary layer around the inner surface of the conduit.

The inhaler according to the present invention is operated as follows. A user first completely exhales and then inserts the portion 62 into the mouth with the lips closed around the portion 62, and then begins to inhale, establishing air flow from the air tube 34 and establishing the circumferential-swirling, turbulent air flow 61 from the vortex generators 54. Once these air flows are established and while continuing to inhale, the user depresses the canister 18 to discharge a metered volume of medication and propellant mixture from the nozzle discharge orifice 30.
The air flowing from the air tube 34 impinges on the plume of aerosolized medication exiting the exit orifice at the apex of the end wall, slowing it down and evaporating most of the aerosol propellent. The air flow 61 generated by the vortex generators primarily keeps the inner conduit walls free of medication particles while also promoting mixing of the medication with air and evaporating the remainder of the propellant not evaporated by the impinging jet of air from the air tube 34. The user continues to inhale to fill the lungs to their capacity, and then typically holds the breath for a period of time to allow the aerosolized medication to settle within the airways of the lungs.

## Claims

1. An aerosol flow control apparatus comprising:
a. a housing (12) including an open end (20) and a conduit (16) having a wall including an inner surface;
b. a medication dispenser disposed within or supported in said housing (12) and adapted to dispense a dose of aerosolized medication into said conduit (16), and
c. a plurality of air inlets (46) in fluid communication with ambient outside air
d. a plurality of vortex generators (54) positioned downstream from said medication dispenser for establishing a circumferential-swirling turbulent air flow along said inner surface upon an air flow being established through said open end
**characterized by**
e. said vortex generators being positioned within said wall and having a plurality of outlets located on said inner surface, wherein said vortex generators (54) are substantially pyramid-shaped having an open base (58) forming said outlet and having an apex in fluid communication with said air inlets (46)

2. The aerosol flow control apparatus according to claim 1,
wherein said conduit (16) defined by a wall including an inner and an outer surface and
said plurality of air inlets (46) is positioned on said outer surface and
said open end is adapted to be inserted into the mouth of a user and said housing comprises a substantially closed end remote from said open end.

3. The aerosol flow control apparatus of claim 2,
wherein the medication dispenser comprises a pressurized canister (18) of medication, an actuator and nozzle assembly including a bore adapted to receive a hollow outlet stem of said canister, and a nozzle discharge orifice in fluid communication with said bore and arranged to direct a plume of aerosolized medication into said conduit (16).

4. The aerosol flow control apparatus according to claim 2 or 3,
wherein the closed end is generally conical or hemispherical in shape with an apex forming a portion of said closed end farthest from said open end of said conduit, and
wherein said air inlets (46) are positioned adjacent the juncture between said closed end and said conduit.

5. The aerosol flow control apparatus of any of claims 2 to 4,
wherein said actuator and nozzle assembly includes a second nozzle discharge orifice in fluid communication with said bore, said two nozzle discharge orifices being spaced apart and oriented at an angle to one another such that the plumes discharged from said orifices impinge on one another within said conduit (46) so as to promote dispersion and mixing of the aerosolized medication.

6. The aerosol flow control apparatus according to any of the preceding,
wherein each said vortex generator (54) comprises a first generally pyramid-like portion (56) having an apex and an open base (58) forming said outlet and a second generally pyramid-like portion having an apex and an open base in fluid communication with a corresponding air inlet, said apexes overlapping (54) to form an opening allowing fluid communication between said portions.

7. The aerosol flow control apparatus according to anyone of the preceding claims,
further comprising an air tube supported within said conduit (16) and having an outlet arranged opposite said nozzle discharge orifice and an inlet in fluid communication with ambient air outside said conduit, wherein an inspiratory effort exerted on the open end of said conduit causes air to flow into said air tube inlet and out of said air tube outlet, said air tube being oriented so that air flowing out of said air tube outlet is directed so as to impinge on a plume of aerosolized medication discharged from the canister (18) through the nozzle discharge orifice.

8. The aerosol flow control apparatus according to anyone of the preceding claims,
wherein said medication dispenser (18) includes a pressurized canister containing medication, and said housing (12) includes an actuator and nozzle assembly adapted to receive a hollow discharge stem of said canister (18), said actuator and nozzle assembly having a nozzle discharge orifice disposed to discharge aerosolized medication into said conduit (16).

9. The aerosol flow control apparatus according to anyone of the preceding claims,
wherein said conduit (16) includes a substantially closed end remote from said open end, said closed end including said plurality of air inlets.

10. The aerosol flow control apparatus according to anyone of the preceding claims,
wherein said conduit (16) is generally tubular.

## Patentansprüche

1. Aerosol-Durchflussregelungsvorrichtung, umfassend:
a. ein Gehäuse (12), welches ein offenes Ende (20) und ein Rohr (16) enthält, welches eine Wand aufweist, welche eine innere Oberfläche enthält;
b. ein Medikamentenspender, welcher innerhalb oder gelagert in dem Gehäuse (12) angeordnet ist und angepasst ist, um eine Dosis eines in Aerosolform vernebelten Medikaments in das Rohr (16) auszugeben, und
c. eine Vielzahl von Lufteinlässen (46), welche in Fluidkommunikation mit äußerer Umgebungsluft sind,
d. eine Vielzahl von Wirbelerzeugern (54), welche stromabwärts von dem Medikamentenspender positioniert sind zum Aufbauen eines umlaufenden wirbelnden turbulenten Luftstroms entlang der inneren Oberfläche auf einen Luftstrom hin, welcher durch das offene Ende aufgebaut wird;
**dadurch gekennzeichnet, dass**
e. die Wirbelerzeuger innerhalb der Wand positioniert sind und eine Vielzahl von Auslässen aufweisen, welche auf der inneren Oberfläche angeordnet sind,
wobei die Wirbelerzeuger (54) im Wesentlichen Pyramidenförmig sind, eine offene Basis (58) aufweisend, welche den Auslass bildet, und welche eine Spitze in Fluidkommunikation mit den Lufteinlässen (46) aufweist.

2. Die Aerosol-Durchflussregelungsvorrichtung gemäß Anspruch 1,
wobei das Rohr (16), welches durch eine Wand definiert wird, welche eine innere und eine äußere Oberfläche und eine Vielzahl von Lufteinlässen (46) enthält, auf der äußeren Oberfläche positioniert ist, und
das offene Ende angepasst ist, um in den Mund eines Benutzers eingeführt zu werden, und das Gehäuse ein im Wesentlichen geschlossenes Ende entfernt von dem offenen Ende umfasst.

3. Die Aerosol-Durchflussregelungsvorrichtung gemäß Anspruch 2,
wobei der Medikamentenspender einen unter Druck stehenden Behälter (18) mit Medikament, eine Aktuator- und Düsenanordnung, welche eine Bohrung enthält, welche angepasst ist, um einen hohlen Auslassschaft des Behälters aufzunehmen, und eine Düsenauslassöffnung in Fluidkommunikation mit der Bohrung und angeordnet, um eine Schwade in Aerosolform vernebelten Medikaments in das Rohr (16) zu leiten, umfasst.

4. Die Aerosol-Durchflussregelungsvorrichtung gemäß Anspruch 2 oder 3,
wobei das geschlossene Ende im Allgemeinen konisch oder halbkugelförmig in der Form ist, wobei eine Spitze einen Abschnitt des geschlossenen Endes am weitesten weg von dem offenen Ende des Rohrs bildet, und wobei die Lufteinlässe (46) angrenzend an der Verbindungsstelle zwischen dem geschlossenen Ende und dem Rohr positioniert sind.

5. Die Aerosol-Durchflussregelungsvorrichtung gemäß einem der Ansprüche 2 bis 4,
wobei die Aktuator- und Düsenanordnung eine zweite Düsenauslassöffnung in Fluidkommunikation mit der Bohrung enthält, wobei die zwei Düsenauslassöffnungen voneinander beabstandet sind und in einem Winkel zueinander orientiert sind, so dass die Schwaden, welche von den Öffnungen ausgegeben werden, in dem Rohr (46) aufeinandertreffen, um eine Dispersion und ein Mischen des in Aerosolform vernebelten Medikaments zu fordern.

6. Die Aerosol-Durchflussregelungsvorrichtung gemäß einem der vorhergehenden Ansprüche,
wobei jeder der Wirbelerzeuger (54) einen ersten im Allgemeinen Pyramidenähnlichen Abschnitt (56) umfasst, welcher eine Spitze und eine offene Basis (58) aufweist, welche den Auslass bildet und einen zweiten im Allgemeinen Pyramidenähnlichen Abschnitt, welcher eine Spitze und eine offene Basis in Fluidkommunikation mit einem entsprechendem Lufteinlass aufweist, wobei sich die Spitzen (54) überlappen, um eine Öffnung zu bilden, welche eine Fluidkommunikation zwischen den Abschnitten erlaubt.

7. Die Aerosol-Durchflussregelungsvorrichtung gemäß irgendeinem der vorhergehenden Ansprüche,
welche weiterhin eine Luftröhre umfasst, welche innerhalb des Rohrs (16) gelagert ist, und welche einen Auslass, welcher gegenüber der Düsenauslassöffnung angeordnet ist, und einen Einlass in Fluidkommunikation mit Umgebungsluft außerhalb des Rohrs aufweist, wobei eine Ansaugkraft, die auf das offene Ende des Rohrs ausgeübt wird, bewirkt, dass Luft in die Lufteinlassröhre und aus dem Luftröhrenauslass heraus fließt,
wobei die Luftröhre derartig orientiert ist, dass Luft, welche aus dem Luftröhrenauslass heraus fließt, derartig geleitet wird, um auf eine Schwade in Aerosolform vernebelten Medikaments zu treffen, welche von dem Behälter (18) durch die Düsenauslassöffnung ausgegeben wird.

8. Die Aerosol-Durchflussregelungsvorrichtung gemäß irgendeinem der vorhergehenden Ansprüche, wobei der Medikamentenspender (18) einen unter Druck stehenden Behälter enthält, welcher das Medikament enthält, und wobei das Gehäuse (17) eine Aktuator- und Düsenanordnung enthält, welche angepasst ist, um einen hohlen Auslassschaft von dem Behälter (18) aufzunehmen, wobei die Aktuator- und Düsenanordnung eine Düsenauslassöffnung aufweist, welche angeordnet ist, um in Aerosolform vernebeltes Medikament in das Rohr (16) auszugeben.

9. Die Aerosol-Durchflussregelungsvorrichtung gemäß irgendeinem der vorhergehenden Ansprüche, wobei das Rohr (16) ein im Wesentlichen geschlossenes Ende entfernt von dem offenen Ende enthält, wobei das geschlossene Ende die Vielzahl von Lufteinlässen enthält.

10. Die Aerosol-Durchflussregelungsvorrichtung gemäß irgendeinem der vorhergehenden Ansprüche, wobei das Rohr (16) im Allgemeinen röhrenförmig ist.

## Revendications

1. Appareil de commande de débit d'aérosol comportant :
a. un logement (12) comportant une extrémité ouverte (20) et un conduit (16) ayant une paroi contenant une surface interne ;
b. un distributeur de médicament disposé ou supporté dans ledit logement (12) et adapté pour distribuer une dose de médicament sous forme aérosol dans ledit conduit (16), et
c. une pluralité d'entrées d'air (46) en communication fluide avec l'air externe ambiant
d. une pluralité de générateurs de tourbillon (54) positionnés en aval dudit distributeur de médicament pour établir un courant d'air turbulent tourbillonnant circonférentiellement le long de ladite surface interne en cas d'établissement de courant d'air à travers ladite extrémité ouverte
**caractérisé en ce que**
e. lesdits générateurs de tourbillon étant positionnés à l'intérieur de ladite paroi et ayant une pluralité de sorties situées sur ladite surface interne, lesdits générateurs de tourbillon (54) sont sensiblement en forme de pyramide avant une base ouverte (58) formant ladite sortie et ayant un sommet en communication fluide avec lesdites entrées d'air (46).

2. Appareil de commande de débit d'aérosol selon la revendication 1,
dans lequel ledit conduit (16) défini par une paroi englobant une surface interne et une surface externe et ladite pluralité d'entrées d'air (46) est positionné sur ladite surface externe et
ladite extrémité ouverte est adaptée pour s'introduire dans la bouche d'un utilisateur et ledit logement comporte une extrémité sensiblement fermée distante de ladite extrémité ouverte.

3. Appareil de commande de débit d'aérosol selon la revendication 2,
dans lequel le distributeur de médicament comporte un flacon de médicament sous pression (18), un actionneur et une buse équipée comportant un alésage adapté pour recevoir une tige de sortie creuse dudit flacon, et un orifice de décharge de buse en communication fluide avec ledit alésage et agencé pour diriger un nuage dudit médicament sous forme aérosol dans ledit conduit (16).

4. Appareil de commande de débit d'aérosol selon la revendication 2 ou 3,
dans lequel l'extrémité fermée est généralement de forme conique ou hémisphérique avec un sommet formant une portion de ladite extrémité fermée la plus éloignée de ladite extrémité ouverte dudit conduit, et dans lequel lesdites entrées d'air (46) sont positionnées de façon adjacente à la jonction entre ladite extrémité fermée et ledit conduit.

5. Appareil de commande de débit d'aérosol selon l'une quelconque des revendications 2 à 4,
dans lequel ledit actionneur et ladite buse équipée comportent un second orifice de décharge de buse en communication fluide avec ledit alésage, lesdits deux orifices de décharge de buse étant espacés et orientés l'un par rapport à l'autre en décrivant un angle de sorte que les nuages déchargés desdits orifices empiètent l'un sur l'autre à l'intérieur dudit conduit (46) pour favoriser la dispersion et le mélange du médicament sous forme aérosol.

6. Appareil de commande de débit d'aérosol selon l'une quelconque des revendications précédentes,
dans lequel chaque générateur de tourbillon (54) comporte une première portion généralement en forme de pyramide (56) ayant un sommet et une base ouverte (58) formant ladite sortie, et une seconde portion généralement en forme de pyramide ayant un sommet et une base ouverte en communication fluide avec une entrée d'air correspondante, lesdits sommets (54) se chevauchant pour former une ouverture permettant une communication fluide entre lesdites portions.

7. Appareil de commande de débit d'aérosol selon l'une quelconque des revendications précédentes,
comportant en outre un tube d'air supporté dans ledit conduit (16) et ayant une sortie opposée audit orifice de décharge de buse et une entrée en communication fluide avec l'air ambiant à l'extérieur dudit conduit, dans lequel un effort d'inspiration exercé sur l'extrémité ouverte dudit conduit provoque un écoulement d'air dans ladite entrée de tube d'air et hors de ladite sortie de tube d'air, ledit tube d'air étant orienté pour que l'air s'écoulant hors de ladite sortie de tube d'air soit dirigé pour empiéter sur un nuage de médicament sous forme aérosol refoulé depuis le flacon (18) à travers l'orifice de décharge de buse.

8. Appareil de commande de débit d'aérosol selon l'une quelconque des revendications précédentes,
dans lequel ledit distributeur de médicament (18) comporte un flacon sous pression contenant un médicament, et ledit logement (12) contient un actionneur et une buse équipée adaptés pour recevoir une tige de décharge creuse dudit flacon (18), ledit actionneur et ladite buse équipée ayant un orifice de décharge de buse disposé pour décharger ledit médicament sous forme aérosol dans ledit conduit (16).

9. Appareil de commande de débit d'aérosol selon l'une quelconque des revendications précédentes,
dans lequel ledit conduit (16) comporte une extrémité sensiblement fermée distante de ladite extrémité ouverte, ladite extrémité fermée comportant ladite pluralité d'entrées d'air.

10. Appareil de commande de débit d'aérosol selon l'une quelconque des revendications précédentes,
dans lequel ledit conduit (16) est généralement tubulaire.
